# EUROPEAN PATENT SPECIFICATION

(11) **EP 3 475 705 B1**
(45) Date of publication and mention of the grant of the patent: **17.02.2021**
(21) Application number: 17734438.9
(22) Date of filing: 23.06.2017
(51) Int. Cl.: G01N 21/3577, G01N 33/574

(54) **ANALYSIS OF BODILY FLUIDS USING INFRARED SPECTROSCOPY FOR THE DIAGNOSIS AND/OR PROGNOSIS OF CANCER**
ANALYSE VON KÖRPREFLÜSSIGKEITEN MITTELS INFRAROTSPEKTROSKOPIE ZUR DIAGNOSE UND/ODER PROGNOSE VON KREBS
ANALYSE DES FLUIDES CORPORELS PAR SPECTRSCOPIE INFRAROUGE POUR LE DIAGNSOTIC ET/OU LE PRONOSTIC DU CANCER

(30) Priority: 24.06.2016 GB 201611057
(43) Date of publication of application: 01.05.2019
(73) Proprietor: University of Strathclyde, Glasgow G1 1XQ (GB)
(72) Inventor: BAKER, Matthew J, Glasgow G1 1RD (GB); SPALDING, Katie, Glasgow G1 1RD (GB); HUGHES, Caryn, Glasgow G1 1RD (GB); PALMER, David, Glasgow G1 1RD (GB); SMITH, Benjamin, Glasgow G1 1RD (GB); BONNIER, Franck, 37020 Tours Cedex (FR); BUTLER, Holly Jean, Glasgow G1 1RD (GB)
(74) Representative: Chapman, Paul Gilmour
(86) International application number: PCT/GB2017/051847
(87) International publication number: WO 2017/221027

(56) References cited:
- WO-A1-2014/076480
- WO-A1-2016/061613
- US-A1- 2007 292 963
- LASCH P ET AL: "ANTEMORTEM IDENTIFICATION OF BOVINE SPONGIFORM ENCEPHALOPATHY FROM SERUM USING INFRARED SPECTROSCOPY", ANALYTICAL CHEMISTRY, AMERICAN CHEMICAL SOCIETY, US, vol. 75, no. 23, 1 December 2003 (2003-12-01), pages 6673-6678, XP001047356, ISSN: 0003-2700, DOI: 10.1021/AC030259A
- SALLY CAINE ET AL: "The application of Fourier transform infrared microspectroscopy for the study of diseased central nervous system tissue", NEUROIMAGE, ELSEVIER, AMSTERDAM, NL, vol. 59, no. 4, 9 November 2011 (2011-11-09), pages 3624-3640, XP028456620, ISSN: 1053-8119, DOI: 10.1016/J.NEUROIMAGE.2011.11.033 [retrieved on 2011-11-18]
- K. V Oliver: "Infrared spectroscopy as a clinical diagnostic method for detection of disease states: developments and applications in kidney diseases and cancer diagnoses", , 28 December 2015 (2015-12-28), XP055645580, Retrieved from the Internet: URL:https://discovery.ucl.ac.uk/1473224/1/ K_Oliver_Thesis.pdf [retrieved on 2019-11-22]
- Heinz Fabian ET AL: "Analysis of biofluids in aqueous environment based on mid-infrared spectroscopy", Journal of biomedical Optics, vol. 10, no. 3, 1 January 2005 (2005-01-01), page 031103, XP055645605, ISSN: 1083-3668, DOI: 10.1117/1.1917844
- STOHR E ET AL: "Quantitative FTIR spectrophotometry of cholesterol and other blood constituents and their interference with the in-vitro measurement of blood glucose", BIOENGINEERING CONFERENCE, 1992., PROCEEDINGS OF THE 1992 EIGHTEENTH I EEE ANNUAL NORTHEAST KINGSTON, RI, USA 12-13 MARCH 1992, NEW YORK, NY, USA,IEEE, US, 12 March 1992 (1992-03-12), pages 105-106, XP010067768, DOI: 10.1109/NEBC.1992.285974 ISBN: 978-0-7803-0902-9

## Description

### FIELD

The present invention relates to methods of diagnosing and/or prognosing abnormal conditions, such as proliferative disorders, especially brain cancers, such as gliomas.

### BACKGROUND

Proliferative disorders, such as cancer, are caused by uncontrolled and unregulated cellular proliferation and can, in some cases, lead to the formation of a tumour.

A variety of different imaging techniques can be used to clinically identify tumours, such as computed tomography (CT), magnetic resonance imaging (MRI), X-Rays, and positron emission tomography (PET). However, these techniques can be costly and/or time-consuming. In addition a final biopsy is often required to determine whether any identified tumours are malignant or benign.

It is therefore desirable to identify new methods of diagnosing proliferative diseases, such as tumours, which can be performed quickly and effectively without the initial need for an invasive procedure, which can be problematic in any case depending on the location of any tumour.

Recently attention has turned to the use of IR spectroscopy in the analysis of biofluids to provide useful diagnostic tests. However, the presence of water can pose a problem when attempting IR spectroscopic analysis of biofluids, such as blood samples. Water is a very IR active material and so the intensity of the peaks associated with water often dominate the spectrum of a biofluid and, in many cases, these peaks overlay the peaks of the biofluid spectra which are of interest to the clinician. In order to address this, samples are generally dried prior to analysis being conducted.

Recently a method of diagnosing brain cancer by performing Attenuated Total Reflection - Fourier Transform Infrared (ATR-FTIR) spectroscopic analysis of blood samples has been described in WO 2014/076480). In contrast to conventional ATR-IR (where a sample is placed on a substrate that is then brought into contact with the ATR crystal), the ATR crystal was used as the substrate for the sample. This method provides a point of care and non-destructive diagnostic test. However, it requires careful preparation of the blood samples prior to carrying out a spectroscopic analysis, which included a drying step to minimise the water present in the sample.

P. Lasch et al (Anal Chem 2003, 75, 6673-6678) describes analysis of the IR spectral signature of blood serum samples in a method of diagnosing BSE and similarly, WO2016/061613 describes detecting an infectious agent.

US2007/0292963, S. Caine et al (Neurolmage 59 (2011), 3624-3640) and K.V. Oliver (UCL doctoral thesis 2015)) are all concerned with the diagnosis of proliferative diseases, but do not describe using IR spectroscopy, or liquid blood serum samples.

H. Fabian et al (J. Biomedical Optics 2005, 10(3), 031103) and E. Stohr et al (Department of Biomedical Engineering, Worcester Polytechnic Institute (1992)) describe methods which are based on the analysis of FTIR spectra obtained from liquid blood samples, but do not describe diagnosing proliferative diseases, such as cancer.

It is an object of the present invention to obviate and/or mitigate at least one of the aforementioned disadvantages.

### SUMMARY

The present invention is based on the surprising discovery that spectroscopic analysis of wet biofluids can assist in the diagnosis and/or prognosis of abnormal conditions, such as proliferative diseases, such as cancer. The techniques described herein facilitate rapid diagnosis of proliferative diseases.
1. According to an aspect there is provided a method of diagnosing and/or
   prognosing a proliferative disorder in a subject, the method comprising: performing an ATR-IR spectroscopic analysis of a liquid blood serum sample from the subject to produce a spectroscopic signature characteristic of the liquid blood serum sample, wherein the liquid blood serum sample has been coated onto a surface of an ATR crystal and wherein the spectroscopic signature characteristic of the liquid blood serum sample is compared to a database of representative signatures previously obtained from samples from subjects with known proliferative or non-proliferative disorders, in order to diagnose and/or prognose, through comparison of the respective signatures, whether or not the subject has a proliferative disorder, such as cancer.

The spectroscopic signature characteristic of the biofluid/blood sample is compared to a database of representative signatures previously obtained from samples from subjects with known abnormalities, such as proliferative or non-proliferative disorders, in order to diagnose and/or prognose, through comparison of the respective signatures, whether or not the subject has an abnormality, such as a proliferative disorder, such as cancer. Such comparison would be carried out using pattern recognition software and/or machine learning analysis known in the art. Alternatively clinical measures such as sugar, carbohydrate, protein and nucleic acid, metabolites and lipid levels can be quantified and used as markers of abnormalities/cell proliferation. For example, total albumin and/or globulin levels may be determined from the biofluid/blood sample, such as a serum sample.

As used herein, a "wet" biofluid/blood sample refers to a sample which has not been dried prior to spectroscopic analysis. In the claimed methods, the wet blood sample is a liquid blood serum sample. As such, the sample may contain water (e.g. up to 95% water) and/or be in liquid form. The inventors have surprisingly identified that spectroscopic analysis of such wet biofluid samples can provide a rapid indication of the presence of an abnormality, such as a proliferative disease in a subject. Thus, unlike prior art processes, the present methods do not require the liquid sample to be dried prior to IR spectroscopic analysis being carried out. In some embodiments of the present invention the method is conducted on the liquid sample quickly, before the sample may be allowed to dry. Thus, the method may be conducted, for example, within 1 minute, 5 minutes, or 10 minutes of the sample being applied to a substrate for detection.

The method may provide an indication of whether a subject is cancerous or non-cancerous/benign. In other instances, the method may provide a more specific diagnosis of a particular condition or proliferative disease.

According to a further aspect there is provided a method of diagnosing and/or prognosing a proliferative disorder in a subject according to the first aspect, comprising: analysing the spectroscopic signature to determine whether a proliferative disorder is present;
where a proliferative disorder is present, further analysing the spectroscopic signature, or obtaining a second ATR-IR spectroscopic signature characteristic of the liquid blood serum sample; and
analysing the signature further or the second spectroscopic signature to provide a more specific diagnosis of the proliferative disorder.

As will be appreciated, a two-stage method as described above, may provide an early and rapid indication of whether or not a proliferative disease, such as brain cancer, is present in a subject. If the first spectroscopic analysis indicates that such a proliferative disease is present, then a secondary analysis may provide a more specific diagnosis (e.g. primary brain vs secondary brain tumours, or benign tumours).

In some cases, the method may comprise performing a second spectroscopic analysis of a dried biofluid/blood sample (or component thereof) to obtain the second spectroscopic signature. In such cases, both the first and second spectroscopic analysis may be carried out on the same instrument.

Alternatively, the method may comprise further processing of the first spectroscopic signature carried out on a wet sample, so as to compare the first spectroscopic signature with databases of spectra obtained from wet samples from subjects with specific proliferative conditions. Such further processing may be to comparing the signature obtained from the subject to a database of signatures previously obtained from dried biofluid/blood samples from subjects with known proliferative/non-proliferative disorders. Such comparison would be carried out using pattern recognition software and/or machine learning analysis known in the art.

For example the signature obtained from a subject may be compared with a database of signatures which allow differentiation between an abnormality/proliferative disorder positive and negative subjects. Only if a subject is identified as being abnormal or being proliferation disorder positive will the further analysis be carried out to seek to further ascertain more specifically the type of the abnormality or the proliferative disorder, such as a primary tumour, or metastatic tumour.

In other cases, the second spectroscopic signature may be obtained by digitally drying a spectrum obtained from the first spectroscopic analysis of the wet blood sample. As used herein, "digitally drying" a spectrum may comprise a software-based least squares method to "digitally" remove water peaks from a spectrum.

The methods of the invention generally use infra-red (IR) spectroscopic analysis. The spectroscopic signature characteristic of the blood sample (which may be referred to as the signature or fingerprint region) may be part or all of the relevant IR spectrum between 400 to 2500 cm⁻¹, part or all of the spectrum between 800 and 2000 cm⁻¹, or alternatively the spectrum between 900 and 1800 cm⁻¹, in addition areas within the whole spectrum from 400 - 4000 cm⁻¹ may be relevant

Prior to a spectroscopic analysis, a background spectrum may be obtained. Such background spectra may provide correction for a background environment. For example, the background spectrum of air may be obtained to provide an atmospheric correction. Alternatively, or additionally, the background spectrum of a solution may be obtained, e.g. an aqueous solution such as phosphate buffered saline (PBS).

The spectroscopic analysis may involve normalisation (e.g standard normal variate), noise reduction (e.g. PCA based) and derivatisation (e.g. 1^{st} or 2^{nd}), as pre-processing steps.

Various IR techniques that may be employed in the methods of this disclosure are described below.

The method may use Fourier transform IR (FTIR) spectroscopic analysis. In FTIR, the IR spectra may be collected in the region of 400-4000 wavenumbers (cm⁻¹). Generally the IR spectra may have a resolution of 10 cm⁻¹ or less, 5 cm⁻¹ or less, or approximately 4 cm⁻¹. The FTIR spectroscopic analysis may employ at least 10 scans, at least 15, or at least 30 scans. The FTIR spectroscopic analysis may employ at most 100 scans, at most 50 scans, or at most 40 scans. For example, 32 scans may be used. The scans may be co-added. As will be appreciated by the skilled person, the number of scans may be selected to optimize data content and data-acquisition time.

Alternatively, the methods of this disclosure may use discrete frequency IR (DFIR). For instance, a Quantum Cascade Laser (QCL) source may be used. QCLs are semiconductor lasers that emit in the mid to far-infrared portion of the electromagnetic spectrum. QCLs generally provide spectral power densities that are several orders of magnitude higher than globars (Alcaraz et al. Anal Chem, 2015, 87). Using these sources within the regions of interest of a biofluid, such as a blood sample, (e.g. 1800 - 1000 cm⁻¹) enables a deeper penetration of the light into an analyte. Thus, using a QCL in the methods of the invention may enable a greater proportion of the sample to be analysed, thus increasing the sensitivity of the technique. In addition, it is possible to engineer a QCL emitting at a desired wavelength region, e.g. such sources can be tuned to emit at a desired wavelength region. This may allow a spectroscopic signature to be obtained more rapidly by targeting a region of interest (such as a signature region).

The claimed methods use Attenuated Total Reflection (ATR)-IR spectroscopic analysis. In some embodiments, the spectroscopic analysis may be ATR-FTIR.

It was previously believed that the low emission intensity of the classical low intensity IR sources (e.g. a globar) limits the scope for analysing samples with a highly absorbing matrix, e.g. water. In such samples, the water absorbs a large part of the irradiated light, thus attenuating the light intensity which is available for interaction with the actual analyte. As such, in the area of interest for most biofluid research (1800 - 100 cm⁻¹), the thickness of a biofluid analyte was generally limited to 8 micron when performing transmission IR spectroscopy (Alcaraz et al. Anal Chem, 2015, 87). Analogously, it was believed that the intensity of water peaks would also overshadow useful information when performing Attenuated Total Reflection (ATR) IR spectroscopy of water-containing biofluids. In particular, as ATR only penetrates to a depth of approximately 2 micron into the sample, it was thought that this technique would lack sensitivity due to the relatively low concentration of biomolecules (compared to water). However, the present inventors surprisingly identified that an (ATR)-IR spectroscopic analysis of a wet blood sample can provide useful diagnostic information indicating the presence of a proliferative or other disease in a subject. As such, a method involving the use of ATR-IR spectroscopic analysis has been found to be particularly effective in a two-stage method of diagnosis.

Alternatively, the second spectroscopic signature may instead be obtained by digitally drying the first spectroscopic signature.

In a method involving ATR-IR, "ATR crystals" may be employed to support the blood sample during IR analysis. Suitably, the blood sample coats the surface (or part thereof) of the "ATR crystal" during IR analysis. Suitable ATR crystals include germanium, KRS-5 zinc selenide, diamond and silicon. The ATR crystals may be in a plate-like form. In a particular embodiment, the ATR crystal is a single reflection diamond crystal.

During a typical ATR-IR spectroscopic analysis, the blood sample may be loaded onto an ATR crystal and IR light may travel through the ATR crystal, and reflect (e.g. via total internal reflection) at least once off an internal surface of the crystal that is in contact with the sample. Such reflection may form an "evanescent wave" which penetrates into the blood sample to an extent depending on the wavelength of light, the angle of incidence and the indices of refraction for the ATR crystal and the blood sample itself. Reflection numbers can be altered by varying the angle of incidence. The beam may be received by an IR detector as it exits the crystal. The ATR crystal is generally an optical material with a higher refractive index than the blood sample to enable the evanescent wave effect.

The spectroscopic analysis may be IR transmission analysis. During a typical transmission analysis, a sample, such as a blood sample, may be placed on an IR transparent substrate or within an IR transparent medium. IR light may be transmitted through the sample and the resultant beam detected.

In some embodiments, the spectroscopic analysis may be QCL-IR transmission analysis. In such methods, the blood sample may be placed into a transmission cell.

After obtaining a spectroscopic signature characteristic of the blood sample (or component thereof) using one or more of the techniques described above, the signature may then be correlated with a favourable or unfavourable diagnosis and/or prognosis. For example, the signature may be correlated to provide an indication of whether or not a subject is cancerous or non-cancerous. Additionally, or alternatively, the signature may be correlated to provide a diagnosis of a primary or secondary brain cancer.

In particular, the method of diagnosing and/or prognosing an abnormality, such as a proliferative disorder in a subject, may further comprise comparing and/or correlating the spectroscopic signature of the blood sample (or component thereof) to one or more reference spectroscopic signatures indicative of one or more specific abnormalities or proliferative diseases.

The reference spectroscopic signature may be a spectroscopic signature that has previously been correlated with a particular diagnosis. For example, the reference spectroscopic signature may be a pre-correlated signature, optionally stored in a database with other pre-correlated signatures. The reference spectroscopic signature may have been obtained using the same spectroscopic technique and under the same conditions as the spectroscopic signature to be correlated. For example, a spectroscopic signature obtained from a wet blood sample may be correlated with a reference spectroscopic signature also obtained from a wet blood sample.

The spectroscopic signature of the blood sample may typically be in the region of 900-1800 cm⁻¹.

The step of correlating may be undertaken by comparing the spectroscopic signature with one or more pre-correlated signatures (e.g. signatures previously obtained and verified, e.g. via biopsies, as indicative of a particular diagnosis and/or prognosis). This can be achieved by way of a qualitative assessment - e.g. certain signatures will resemble, perhaps to varying degrees, a signature characteristic of a blood sample of a subject with a proliferative disorder, whilst other signatures may differ to such signatures. As such, a qualitative assessment of the appearance of the signature may be used in the diagnosis and/or prognosis of a proliferative disorder. Such a qualitative assessment may be conducted manually, but is preferably conducted digitally via a computer running pursuant of computer software that performs such an assessment. Suitably any such computer software is able, from the assessment, to correlate the signature with a favourable or unfavourable diagnosis and/or prognosis.

Alternatively or additionally, correlation with a particular diagnosis and/or prognosis may be made via a quantitative assessment - e.g. where the biofluid/blood sample signature is compared to one or more reference signatures (already previously correlated with a favourable or unfavourable diagnosis and/or prognosis), optionally stored in a database, and suitably statistically analysed for a likelihood of a correlation.

The method of using a quantitative assessment to assist in diagnosis and/or prognosis would allow the invention described to have an embodiment that is comparable to the blood test taken within the clinical appointment. In particular, the method details how the identification of protein concentration in a biofluid/blood sample through spectroscopic analysis could be compared to those results obtained from a blood test at a routine clinical appointment.

Spectroscopic analysis of standardised or prepared spiked human biofluid samples may be used to produce calibration models using a partial least squares regression (PLSR) algorithm. The spiked samples may be prepared using, for example, two commonly encountered proteins (human serum albumin and gamma-globulins) and concentrations prepared in a range covering expected normal human concentrations. Pre-correlated signatures may be used to carry out a cross validation, testing the validity of the model before they are used to predict the unknown protein concentration in patient samples. In a particular embodiment, a spectroscopically obtained signature is compared to a plurality of pre-correlated signatures stored in a database (e.g. a "training set") in order to derive a correlation with a favourable or unfavourable diagnosis and/or prognosis. Statistical analysis (e.g. via pattern recognition algorithms and machine learning) may be performed, preferably based on a comparison of the similarities and dissimilarities of the signature with the pre-correlated signatures, before the statistical analysis is used to correlate the signature with a favourable or unfavourable diagnosis and/or prognosis. Suitably pattern recognition and machine learning algorithms include support vector machines (SVM), random forest classifiers and principal component discriminant function analysis (PC- DFA).

In a particular embodiment, a spectroscopically obtained signature is correlated with a favourable or unfavourable diagnosis and/or prognosis based on a predictive model developed by "training" (e.g. via pattern recognition algorithms) a database of pre-correlated analyses.

Examination and/or comparison of blood sample signatures from spectroscopic analysis does not necessarily focus on particular peaks, or particular substances responsible for any particular peaks. However, in the case of ATR-FTIR, two amide peaks, which generally appear as a doublet of peaks at approximately 1550 cm⁻¹ and 1650 cm⁻¹ appear to be important indicators of proliferative disorders since certain changes in these peaks indicate changes in protein structure suggestive of a proliferative disorder.

In the methods of the invention, the spectroscopic analysis is carried out on a blood serum sampleln some embodiments, the blood sample may be human blood serum. In some instances, the blood sample may be whole serum. In other cases, the blood sample may be whole serum with components above 100 kDa removed by centrifugation filtration, or whole serum with components above 10 kDa removed by centrifugation filtration, or whole serum with components above 3 kDa removed by centrifugation filtration. Other potential biofluids described in accordance with the present disclosure include, urine, cerebrospinal fluid, faeces, bile, breast milk, ejaculate, mucus, saliva, vitreous humour, tears, lymph and the like.

Any such centrifugal filtrations may be performed using a mini centrifuge combined with appropriate Protein filters at 14,000 rpm as per manufacturer's instructions (e.g. Amicon membrane filters, Merck Millipore).

In some embodiments of the disclosure, a plurality of spectroscopic analyses are performed using a variety of serums/biofluids (i.e. with differing degrees of filtration) derived from the same biofluid/whole blood sample, and the results compared and/or used to cross-validate.

An individual aliquot may be taken from the bulk blood sample and used for each spectroscopic analysis. In this manner, further aliquots can be later used for further spectroscopic analyses on the same blood sample, thereby assisting validation of results. Suitably, at least two spectroscopic analyses are performed on each blood sample. Moreover, suitably each individual spectroscopic analysis is repeated at least twice with the same aliquot, preferably at least three times, to help validate results.

Additionally, or alternatively multiple samples may be obtained at the same time or at different time points and analysed.

The methods of the invention provide an indication of whether or not a subject is suffering from a cancer, e.g. a cancer of the brain or spine (and/or associated tumours). In some embodiments, the proliferative disorder may be a brain cancer. The proliferative disease may be glioma.

Alternatively, or additionally, the methods of the invention may enable the nature of the proliferative disorder to be determined e.g. primary brain cancer or secondary brain cancer (metastatic brain cancer).

The three main types of malignant glioma are astrocytomas, ependymomas (WHO Grade II) and oligodendrogliomas. The diagnostic methods of the invention may apply to all these types of glioma. A tumour with a mixture of the histological features present in the main three is known as a mixed glioma, which the present invention may also serve to diagnose. The table below shows the sub-types of high grade and low-grade gliomas.

| **General Tumour Grade** | **WHO Grade** | **Grade Sub-type** |
|---|---|---|
| **Low Grade** | I | Pilocytic astrocytoma |
| | II | Oligodendroglioma |
| | II | Astrocytoma |
| **High Grade** | III | Anaplastic astrocytomas |
| | III | Oligodendrogliomas |
| | IV | Glioblastoma multiforme |

In a particular embodiment, the brain cancer is either a low grade or high grade glioma. In a particular embodiment, the brain cancer is any one of Pilocytic astrocytoma, Oligodendroglioma, Astrocytoma, Anaplastic astrocytomas, Oligodendrogliomas, Glioblastoma multiforme glioma sub-types. In a particular embodiment, the brain cancer is a Grade III or Grade IV glioma. The brain cancer may, for example, be a Grade IV Glioblastomamultiforme (GBM).

The spectroscopic signature may be correlated with a favourable or unfavourable diagnosis and/or prognosis based on a predictive model developed by "training" (e.g. via pattern recognition and/or machine learning algorithms) a database of pre-correlated analyses.

Correlating the analytical results with a favourable or unfavourable diagnosis and/or prognosis may be performed manually (e.g. by a clinician or other suitable analyst) or automatically (e.g. by computational means). Correlations may be established qualitatively (e.g. via a comparison of graphical traces or signatures) or quantitatively (e.g. by reference to predetermined threshold values or statistical limits). Correlating the analytical results may be performed using a predictive model, optionally as defined herein, which may have been developed by "training" a database of pre-correlated assays and/or analyses.

In a particular embodiment, correlating the analytical results with a favourable or unfavourable diagnosis and/or prognosis involves an initial comparison of the analytical results with a reference standard or with previous analytic results that have already been correlated with a favourable or unfavourable diagnosis and/or prognosis (e.g. pre-correlated analytical results stored in a database). Correlations with previous analytical results may involve a statistical comparison or a "best fit" comparison (e.g. if comparing graphical traces with those stored in a database). The method of correlating the analytical results with a favourable or unfavourable diagnosis and/or prognosis may be a computer-implemented method of correlating. Suitably such computer-implemented methods incorporate predictive models, optionally in conjunction with appropriate databases.

The above described methods may also be used to study the effectiveness of any treatment. Thus, for example, a subject may be analysed before commencing a treatment in order to obtain a first signature and analysed again following a period of treatment in order to ascertain whether or not there has been a change in the signature and hence whether or not a particular treatment may be considered as being effective. Further analyses may be carried out during the course of treatment in order to ensure that the treatment is continuing to be effective.

The methods of the present invention may be used alone or in combination with other methods of detection.

As well as the methods described herein, the present disclosure also extends to a database or databases of representative signatures obtained from wet and/or dried biofluid/blood samples for use in accordance with the present methods

### DETAILED DESCRIPTION

The present invention will now be further described, by way of example only, with reference to the following Figures which show:

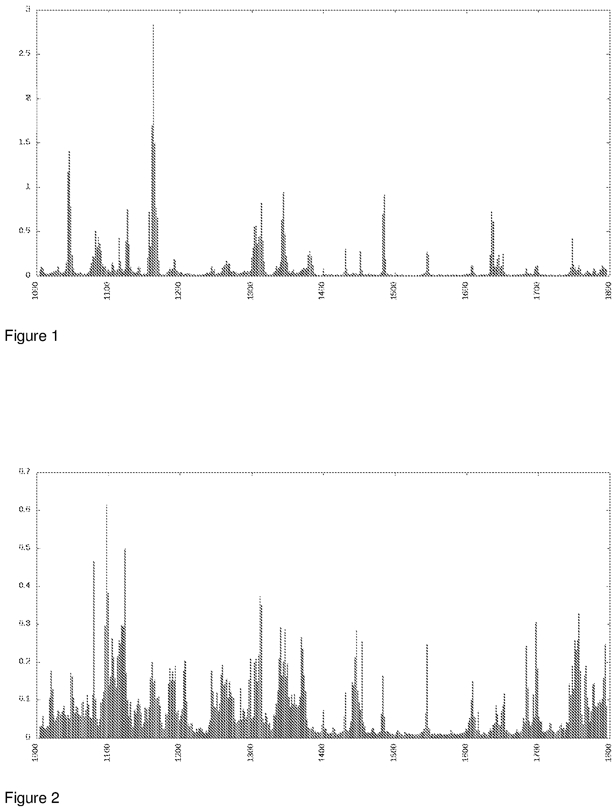

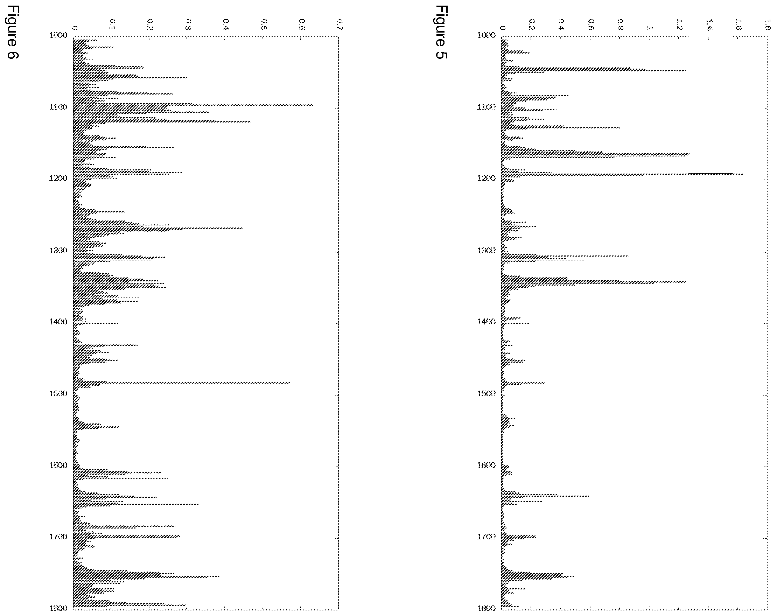

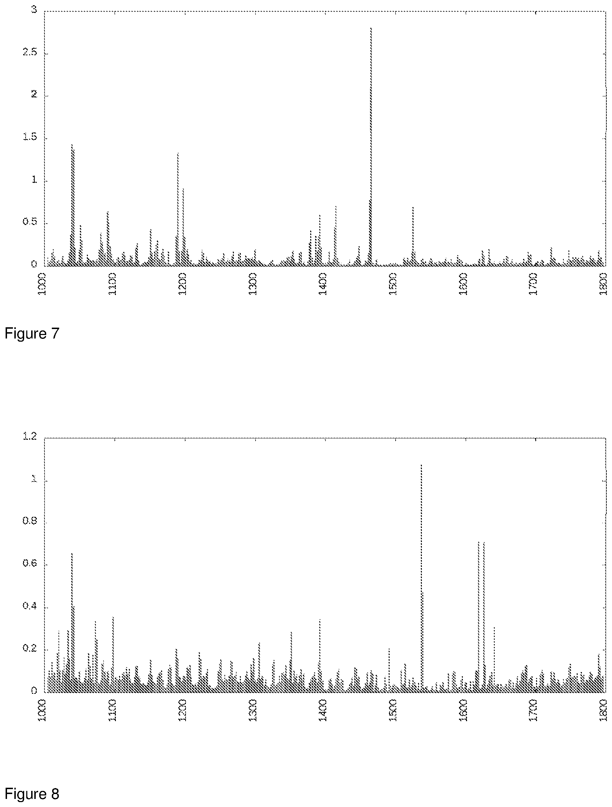

Figures 1 - 12 show the gini coefficient results of various spectra obtained using ATR-FTIR spectral analysis being conducted on wet serum samples and their use in distinguishing between cancer vs non-cancer subjects and subjects with metastatic vs primary glioblastomas.

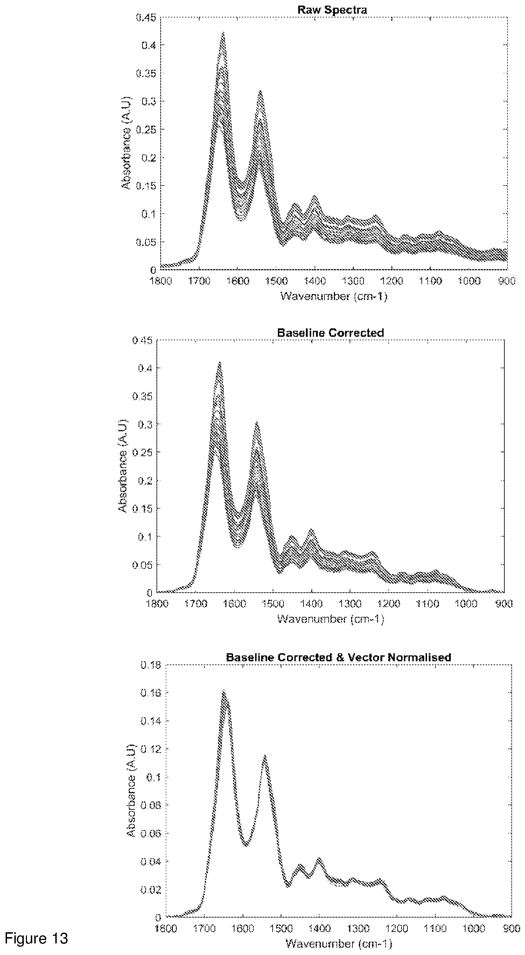

Figures 13 - 17 show the plots following the PLSR of the spiked and patient samples analysed spectroscopically by ATR-FTIR and their use in determining the capability of using this technique to quantify protein concentration in blood samples. In particular: Figure 13 shows plots which display the effect of spectral pre-processing steps. From top to bottom, raw data, baseline corrected data and finally baseline corrected and vector normalised.

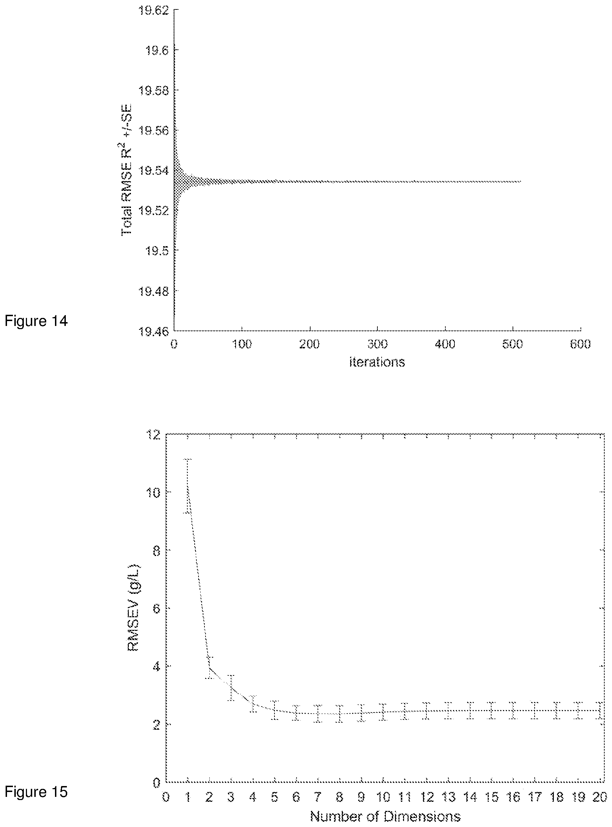

Figure 14 shows a representative convergence plot of the R² value +/- SE vs. the no. of iterations from the 10% air dried globulin analysis. This particular plot led to the selection of 26 iterations which was compared to the number selected from the RMSEC and the RMSEV plots, before the highest value was selected and taken forward to the PLSR analysis
Figure 15 shows evolution of the root mean square error on the validation set (RMSEV). In this case, values are averaged from the 234 cross validations.

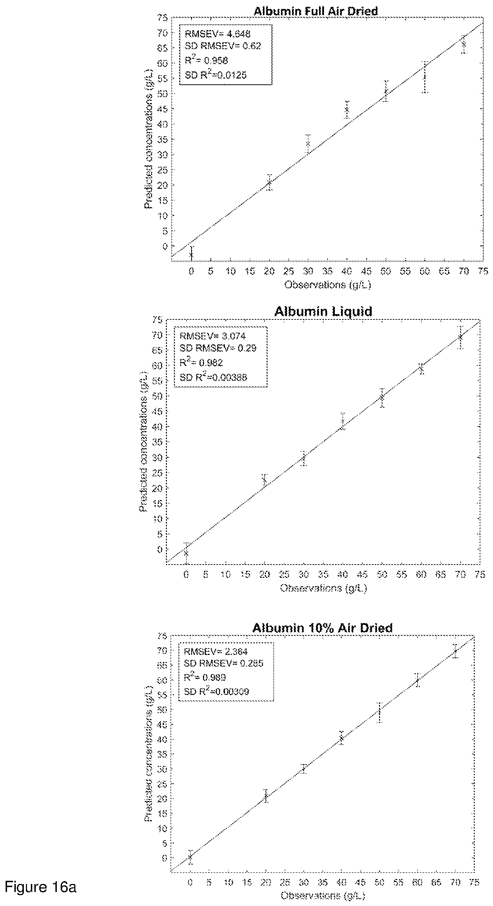

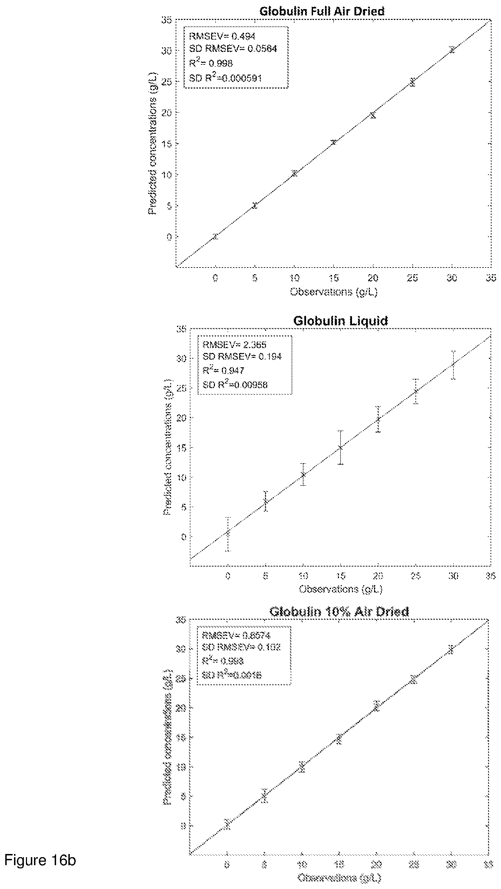

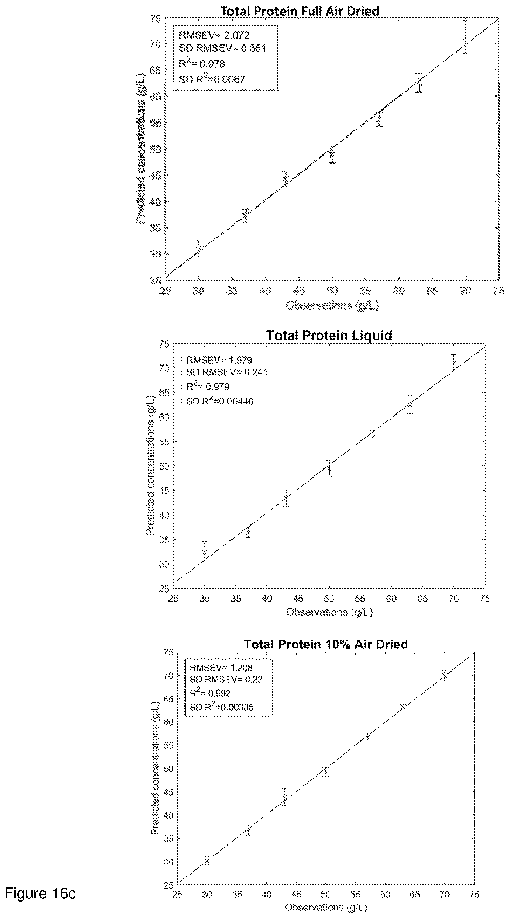

Figures 16a, 16b and 16c show predictive models built from the PLS analysis. Each plot shows the protein used as the spike, as well as the sample state. For each concentration the values displayed are an average of the concentration predicted from the iterations of the corss validation. Shown on each plot is the RMSEV and the R² values as well as the standard deviation corresponding to each of the values.

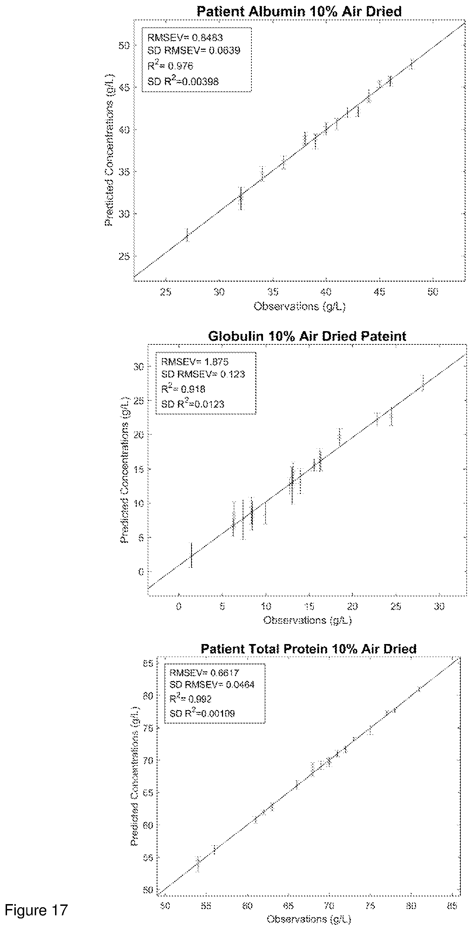

Figure 17 shows predictive models built from the PLS analysis of the patient samples. Each plot depicts a different sample state. For each concentration the values displayed are an average of the concentration predicted from the iterations of the cross validation. Shown on each plot is the RMSEV and the R² values as well as the standard deviation corresponding to each of the values

### EXAMPLES

### Study populations

The dataset used was composed of 150 subjects. The dataset comprised an equal mix of subjects suffering from high grade brain tumours (Grade IV Glioblastomamultiforme (GBM)), metastatic brain cancer (secondary brain cancer from multiple sites within the body) and non-cancerous subjects.
Alternatively, the datasets used for the quantitative measurements consisted of a spiked set made up of samples with concentrations as follows:
Human serum albumin, 7 samples, concentration range 0 mg/ml - 70 mg/ml Immunoglobulin, 7 samples, concentration range 0 mg/ml - 30 mg/ml Combined mixture (total protein), 70 mg/ml - 30 mg/ml

Additionally, a patient set made up of 20 patients with total protein concentrations of 81 mg/ml - 54 mg/ml was predicted using the calibration models from the prepared spiked samples.

### Spectral Collection

1) ATR-FTIR spectral analysis was performed by dropping 1 microlitre of each patient sample onto the surface of the crystal and 3 spectra were collected (from 4000 - 650 or 400 cm⁻¹) in quick succession
   a. In addition for one spectral set the serum was left to dry and 3 spectra were collected again
   b. One spectral set was collected after performing the background to PBS instead of air

### Resulting Datasets

This lead to 4 datasets that we are currently analysing
1) 450 spectra (1 spot of 150 patients analysed 3 times) of wet serum backgrounded to air via ATR-FTIR

### Spectral Data Analysis

Our spectral data analysis process generally follows:
1) Quality test
2) Selection of fingerprint region of the IR spectrum (1800 - 1000 cm-1)
3) Vector normalization followed by either
   a. 1st derivative
   b. 2nd derivative
4) The resulting datasets are split randomly into 2/3 training set and 1/3 blind (test set) based upon patient populations - so that spectra from one patient are either all in the training set or all in the blind set, but not split between both.
5) The training set is fed into a random forest classifier with 96 iterations
6) The peaks which best describe the diagnostic question at hand within the spectrum (i.e cancer vs non-cancer and GBM vs MET) are discovered and then the blind set is projected into the model based upon this information to give diagnostic predictions, from which sensitivity and specificity are calculated
7) In order to discover if there are any differences between the start of spectral collection and the end of spectral collection for those datasets where we have 3 repeats we have ran 3 different spectral combinations
   a. A model based on 150 spectra that is composed form the 1st collected spectrum from each patient
   b. A model based on 150 spectra that is composed from the 3rd (final collected spectrum from each patient)
   c. A model based on 450 spectra representing all the spectra from each patient

So overall we have performed 20 analyses
For dataset 1 above we have
A) 1st spectrum model 1st derivative
B) 1st spectrum model 2nd derivative
C) 3rd spectrum model 1st derivative
D) 3rd spectrum model 2nd derivative
E) All spectra model 1st derivative
F) All spectra model 2nd derivative

We have the same 6 models for dataset 2 and 3 and for the QCL dataset we have 2 analyses (as only 1 spectrum was collected form each patient) that represents the 1st and 2nd derivative analysis

### Results for wet ATR-FTIR analysis

### Cancer vs Non Cancer

The results obtained identify the major peaks that are used in the random forest analysis to discriminate cancer vs non cancer, Figure 1 (or GBM vs Met, Figure 2). The sensitivity and specificity where 84% and 78% respectively for cancer vs non-cancer and 56% and 57% respectively for metastatic vs glioblastoma.

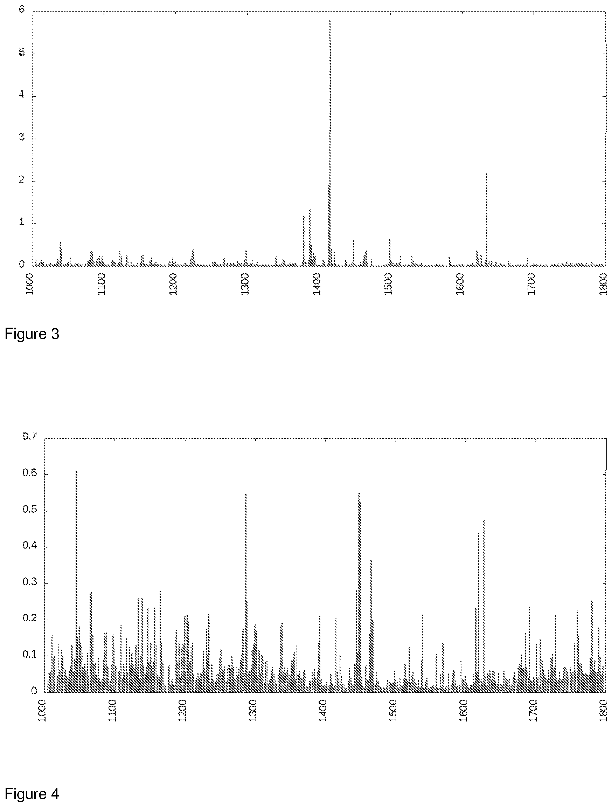

In terms of the 1st spectrum model 2nd derivative for cancer vs non cancer (Figure 3) the sensitivity and specificity were 85% and 82% respectively and for glioblastoma vs metastatic cancer (Figure 4) the sensitivity and specificity were 57% and 59% respectively.

In terms of the 3rd spectrum model 1st derivative for cancer vs non cancer (Figure 5) the sensitivity and specificity were 82% and 77% respectively and for glioblastoma vs metastatic cancer (Figure 6) the sensitivity and specificity were 53% and 54% respectively. The results observed in relation to the 3rd spectrum model 2nd derivative are shown in Figure 7 for cancer vs non cancer (sensitivity and specificity were 78% and 77% respectively) and Figure 8 for glioblastoma vs metastatic cancer (sensitivity and specificity were 58% and 59%.

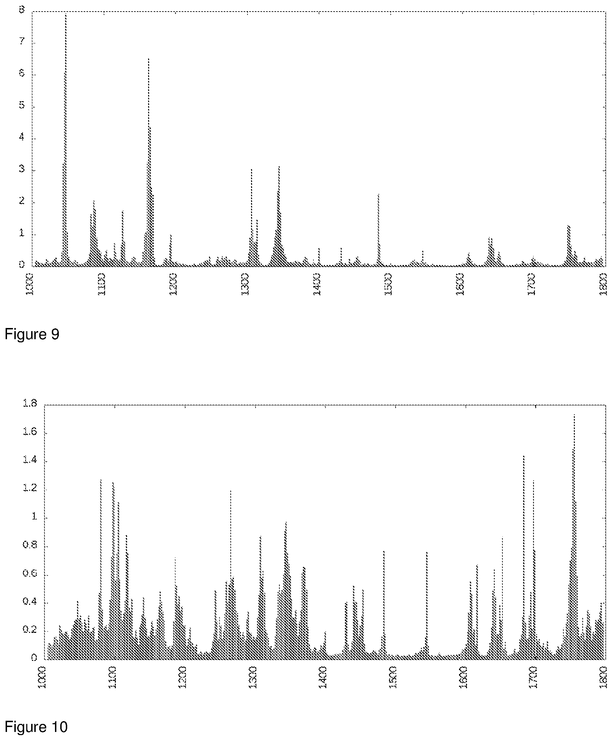

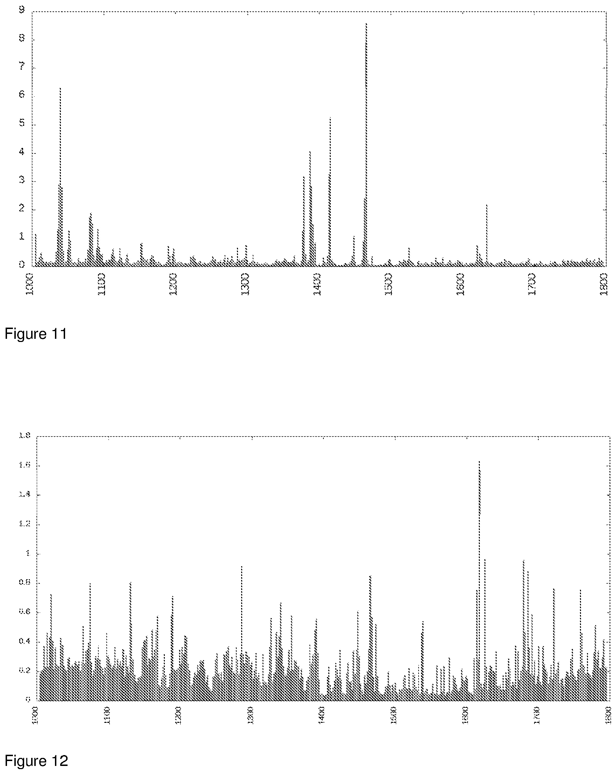

In terms of the all spectrum model 1st derivative for cancer vs non cancer (Figure 9) the sensitivity and specificity were 84% and 78% respectively and for glioblastoma vs metastatic cancer (Figure 10) the sensitivity and specificity were 57% and 58% respectively. The results observed in relation to the all spectrum model 2nd derivative are shown in Figure 11 for cancer vs non cancer (sensitivity and specificity were 84% and 82% respectively) and Figure 12 for glioblastoma vs metastatic cancer (sensitivity and specificity were 56% and 56%.

In conclusion it can be seen that in terms of the use of wet samples, there is a high degree of specificity and sensitivity in terms of distinguishing cancer vs non cancer subjects, although this diminishes somewhat in terms of distinguishing subjects suffering from primary brain tumours (e.g. glioblastoma) as opposed to secondary metastatic brain tumours. Thus, it may be appropriate to conduct a cancer specific detection on dry samples, following an initial detection of a cancer being present being conducted on a wet sample.

### Spectral Collection: Quantitative Measurements

ATR-FTIR spectral analysis was performed by dropping 1 microlitre of each spiked/patient sample onto the surface of the crystal and 3 spectra were collected (from 4000-650cm⁻¹) in quick succession

### Resulting Datasets: Quantitative Measurements

This led to 3 spiked data sets and 1 patient data set
1.) 105 spectra (3 spots of 7 samples analysed 5 times) of spiked samples backgrounded to air via ATR-FTIR
2.) 300 spectra (3 spots of 20 samples analysed 5 times) of 10% diluted air dried patient samples backgrounded to air via ATR-FTIR

### Spectral Data Analysis: Quantitative Measurements

Our spectral data analysis process generally follows:
1.) Selection of the fingerprint region (1800 - 900 cm⁻¹)
2.) Rubberband baseline correction
3.) Vector normalisation
4.) 512 cross validation iterations, 1000 times was carried out on the data, producing three convergence plots, for the outputs of the RMSE calibration (RMSEC), RMSE validation (RMSEV) and R² values. A one term power series model was fitted to the curve highlighting when the gradient was <0.0001 and therefore optimal number of cross validation loops to carry out. However, it meant the number of cross validation loops were not the same over the different proteins, but was consistently selected as the largest
5.) Resulting spiked datasets were split 50:50 calibration:validation to run the PLSR
6.) Mean and standard deviation of the RMSE (measure of the models precision) and R² (measure of the models linearity) were calculated for each spiked data set and used to determine optimal model to use
7.) Once the capability of ATR-FTIR spectroscopy to determine the protein concentration of spiked samples as well as a random patient sample set was identified, the patient set was blindly tested with samples of unknown concentrations.
8.) PLSR was repeated using the spiked model as the calibration set and the patient samples as the validation to blindly test the model

So overall 15 spiked models were produced:
Air Dried = Globulin, Albumin, Total, Total (Glob), Total (Alb)
Air Dried 10% Dilution = Globulin, Albumin, Total, Total (Glob), Total (Alb)
Liquid = Globulin, Albumin, Total, Total (Glob), Total (Alb)

### Results: Quantitative Measurements

From the initial PLSR carried out on the spiked dataset, to determine the optimal sample state (wet, dry or diluted dry), the predictive RMSE and R² values were compared for all 15 models - Figures 16a, 16b, and 16c show graphically the results obtained for Albumin, Globulin and Total Protein.

The Table below shows the results obtained from the 15 models

| | **Liquid** RMSEV ± STD | R² | **Air Dried** RMSEV ± STD | R² | **10% Air Dried** RMSEV ± STD | R² |
|---|---|---|---|---|---|---|
| HSA | 3.074 ± 0.290 | 0.982 | 4.648 ± 0.620 | 0.958 | 2.364 ± 0.285 | 0.989 |
| IgG | 2.365 ± 0.194 | 0.947 | 0.494 ± 0.056 | 0.998 | 0.8574 ± 0.102 | 0.993 |
| Mixed: HSA | 3.429 ± 0.441 | 0.980 | 3.575 ± 0.633 | 0.978 | 2.056 ± 0.356 | 0.992 |
| Mixed:IgG | 1.452 ± 0.179 | 0.980 | 1.503 ± 0.261 | 0.979 | 0.847 ± 0.146 | 0.993 |
| Mixed:Total | 1.979 ± 0.241 | 0.979 | 2.071 ± 0.361 | 0.978 | 1.208 ± 0.220 | 0.992 |

The best RMSEV, highlighting precision, for each model, was consistently obtained from the 10% dried samples, the best being 0.857 ± 0.102 mg/ml from the globulin ATR-FTIR spectra estimating the globulin concentration. This result shows that concentrations can be estimated unambiguously. The R² values, a measure of the models linearity, followed a similar trend, the best results coming from the air dried 10% diluted samples, the optimal result being 0.993.

As the air dried 10% produced the best results from the spiked models, the patient samples were analysed in this way (Figure 17). The predictive values obtained were as follows: for the quantification of total protein concentration a RMSEV of 0.6617 ± 0.0464 and an R² value of 0.992 were achieved.

When quantifying the albumin and globulin concentrations in the patient samples individually, the predictive model performed comparably to the spiked samples. Quantification of the albumin concentration from the air dried diluted samples resulted in a RMSEV of 0.8483± 0.0639 and an R² value of 0.976. Globulin quantification produced an RMSEV of 1.875 ±.0.123 and an R² value of 0.918 which are both relatively close to the spiked samples.

Concluding, this disclosure highlights the ability of infrared spectroscopy to not only detect two different protein molecules but to quantify them combined and individually in within complex biological matrices as spiked human pooled and patient serum samples.

## Claims

1. A method of diagnosing and/or prognosing a proliferative disorder in a subject,
the method comprising:
performing an ATR-IR spectroscopic analysis of a liquid blood serum sample from the subject to produce a spectroscopic signature characteristic of the liquid blood serum sample, wherein the liquid blood serum sample has been coated onto a surface of an ATR crystal and wherein the spectroscopic signature characteristic of the liquid blood serum sample is compared to a database of representative signatures previously obtained from samples from subjects with known proliferative or non-proliferative disorders, in order to diagnose and/or prognose, through comparison of the respective signatures, whether or not the subject has a proliferative disorder, such as cancer.

2. The method according to claim 1 wherein the spectroscopic signature is used to quantify a level of one or more proteins in the liquid blood serum sample in order that the protein level is used as a marker of cell proliferation.

3. The method according to claim 2 wherein said one or more proteins is/are albumin and/or globulin.

4. The method according to any preceding claim, wherein the ATR-IR analysis is carried out between 400 to 2500 cm⁻¹ on the electromagnetic spectrum.

5. The method according to any preceding claim, wherein a background spectrum is obtained in order to provide for correction for a background environment.

6. The method according to any preceding claim, wherein the spectroscopic analysis comprises normalisation, noise reduction and/or derivatisation as one or more pre-processing step(s) and/or Fourier transform IR (FTIR) spectroscopic analysis.

7. A method of diagnosing and/or prognosing a proliferative disorder in a subject according to claim 1, comprising:
analysing the spectroscopic signature to determine whether a proliferative disorder is present;
wherea proliferative disorder is present, further analysing the spectroscopic signature, or obtaining a second ATR-IR spectroscopic signature characteristic of the liquid blood serum sample; and
analysing the signature further or the second spectroscopic signature to provide a more specific diagnosis of the proliferative disorder.

8. The method according to claim 7 further comprising performing a second spectroscopic analysis of a dried blood serum sample to obtain the second spectroscopic signature.

9. The method according to claim 7 wherein the second spectroscopic signature is obtained by digitally drying the spectroscopic signature.

10. The method according to claim 7 wherein further processing of the spectroscopic signature is carried out on the liquid blood serum sample, so as to compare the spectroscopic signature with databases of spectra obtained from liquid blood serum samples from subjects with specific proliferative conditions.

11. The method according to any preceding claim wherein analysis of the signature comprises comparing and/or correlating the spectroscopic signature of the liquid blood serum sample to one or more reference spectroscopic signatures indicative of one or more specificproliferative diseases.

12. The method according to claim 11 wherein the signature is correlated with a favourable or unfavourable diagnosis and/or prognosis based on a predictive model developed by "training" (e.g. via pattern recognition algorithms) a database of pre-correlated analyses.

13. The method according to claims 11 or 12 wherein analysis of the signature provides an indication of whether or not a subject is cancerous or non-cancerous; and wherein the analysis may provide a diagnosis of a primary or secondary brain cancer.

## Patentansprüche

1. Verfahren zum Diagnostizieren und/oder Prognostizieren einer proliferativen Störung bei einem Subjekt,
wobei das Verfahren umfasst:
Durchführen einer ATR-IR-spektroskopischen Analyse einer flüssigen Blutserumprobe vom Subjekt, um eine spektroskopische Signatur zu erzeugen, die für die flüssige Blutserumprobe charakteristisch ist, wobei die flüssige Blutserumprobe auf eine Fläche eines ATR-Kristalls aufgetragen worden ist und wobei die spektroskopische Signatur, die für die flüssige Blutserumprobe charakteristisch ist, mit einer Datenbank repräsentativer Signaturen, die vorab von Proben von Subjekten mit bekannten proliferativen oder nicht proliferativen Störungen erhalten werden, verglichen wird, um, durch Vergleich der jeweiligen Signaturen, zu diagnostizieren und/oder zu prognostizieren, ob das Subjekt eine proliferative Störung, wie z. B. Krebs, hat oder nicht.

2. Verfahren nach Anspruch 1, wobei die spektroskopische Signatur verwendet wird, um einen Spiegel eines oder mehrerer Proteine in der flüssigen Blutserumprobe zu quantifizieren, damit der Proteinspiegel als Marker von Zellproliferation verwendet wird.

3. Verfahren nach Anspruch 2, wobei das eine oder die mehreren Proteine Albumin und/oder Globulin ist/sind.

4. Verfahren nach einem vorstehenden Anspruch, wobei die ATR-IR-Analyse zwischen 400 und 2500 cm⁻¹ auf dem elektromagnetischen Spektrum ausgeführt wird.

5. Verfahren nach einem vorstehenden Anspruch, wobei ein Hintergrundspektrum erhalten wird, um für eine Korrektur für eine Hintergrundumgebung zu sorgen.

6. Verfahren nach einem vorstehenden Anspruch, wobei die spektroskopische Analyse Normalisierung, Rauschreduktion und/oder Ableitungserstellung als einen oder mehrere Vorverarbeitungsschritte und/oder spektroskopische Fourier-Transform-IR-(FTIR) Analyse umfasst.

7. Verfahren zum Diagnostizieren und/oder Prognostizieren einer proliferativen Störung bei einem Subjekt nach Anspruch 1, umfassend:
Analysieren der spektroskopischen Signatur, um zu bestimmen, ob eine proliferative Störung vorliegt;
wo eine proliferative Störung vorliegt, weiteres Analysieren der spektroskopischen Signatur, oder Erhalten einer zweiten ATR-IR-spektroskopischen Signatur, die für die flüssige Blutserumprobe charakteristisch ist; und
weiteres Analysieren der Signatur oder Analysieren der zweiten spektroskopischen Signatur, um eine spezifischere Diagnose der proliferativen Störung zu erstellen.

8. Verfahren nach Anspruch 7, ferner umfassend Durchführen einer zweiten spektroskopischen Analyse einer getrockneten Blutserumprobe, um die zweite spektroskopische Signatur zu erhalten.

9. Verfahren nach Anspruch 7, wobei die zweite spektroskopische Signatur durch digitales Trocknen der spektroskopischen Signatur erhalten wird.

10. Verfahren nach Anspruch 7, wobei weiteres Verarbeiten der spektroskopischen Signatur an der flüssigen Blutserumprobe ausgeführt wird, um die spektroskopische Signatur mit Datenbanken von Spektren zu vergleichen, die von flüssigen Blutserumproben von Subjekten mit spezifischen proliferativen Zuständen erhalten werden.

11. Verfahren nach einem vorstehenden Anspruch, wobei Analyse der Signatur Vergleichen und/oder Korrelieren der spektroskopischen Signatur der flüssigen Blutserumproben mit einer oder mehreren spektroskopischen Referenzsignaturen umfasst, welche Indikatoren für eine oder mehrere spezifische proliferative Erkrankungen sind.

12. Verfahren nach Anspruch 11, wobei die Signatur mit einer günstigen oder ungünstigen Diagnose und/oder Prognose auf Grundlage eines Vorhersagemodells korreliert wird, das durch "Training" (z. B. *via* Mustererkennungsalgorithmen) einer Datenbank präkorrelierter Analysen entwickelt wird.

13. Verfahren nach Anspruch 11 oder 12, wobei Analyse der Signatur ein Anzeichen dafür liefert, ob ein Subjekt kanzerös oder nicht kanzerös ist oder nicht; und wobei die Analyse eine Diagnose eines primären oder sekundären Hirnkrebses liefern kann.

## Revendications

1. Procédé pour le diagnostic et/ou le pronostic d'un trouble prolifératif chez un sujet,
le procédé comprenant :
la réalisation d'une analyse spectroscopique ATR-IR d'un échantillon de sérum de sang liquide provenant du sujet pour produire une signature spectroscopique caractéristique de l'échantillon de sérum de sang liquide, dans laquelle l'échantillon de sérum de sang liquide a été revêtu sur une surface d'un cristal ATR et dans laquelle la signature spectroscopique caractéristique de l'échantillon de sérum de sang liquide est comparée à une base de données de signatures représentatives obtenues antérieurement à partir d'échantillons provenant de sujets avec des troubles prolifératifs ou non prolifératifs connus, dans le but de diagnostiquer et/ou de pronostiquer, par la comparaison des signatures respectives, si oui ou non le sujet présente un trouble prolifératif, tel qu'un cancer.

2. Procédé selon la revendication 1, dans lequel la signature spectroscopique est utilisée pour quantifier un niveau d'une ou de plusieurs protéines dans l'échantillon de sérum de sang liquide dans le but d'utiliser le niveau de protéine comme un marqueur d'une prolifération cellulaire.

3. Procédé selon la revendication 2, dans lequel lesdites une ou plusieurs protéines est/sont une albumine et/ou une globuline.

4. Procédé selon l'une quelconque des revendications précédentes, dans lequel l'analyse ATR-IR est réalisée entre 400 et 2500 cm⁻¹ dans le spectre électromagnétique.

5. Procédé selon l'une quelconque des revendications précédentes, dans lequel un spectre de bruit de fond est obtenu dans le but de fournir une correction pour un environnement de bruit de fond.

6. Procédé selon l'une quelconque des revendications précédentes, dans lequel l'analyse spectroscopique comprend une normalisation, une réduction du bruit et/ou une dérivatisation en tant qu'une ou plusieurs étape(s) de prétraitement et/ou une analyse spectroscopique d'IR à transformée de Fourier (FTIR).

7. Procédé pour le diagnostic et/ou le pronostic d'un trouble prolifératif chez un sujet selon la revendication 1, comprenant :
l'analyse de la signature spectroscopique pour déterminer si un trouble prolifératif est présent;
lorsqu'un trouble prolifératif est présent, l'analyse ultérieure de la signature spectroscopique ou l'obtention d'une deuxième signature ATR-IR caractéristique de l'échantillon de sérum de sang liquide; et
l'analyse ultérieure de la signature ou l'analyse de la deuxième signature spectroscopique pour donner un diagnostic plus spécifique du trouble prolifératif.

8. Procédé selon la revendication 7 comprenant en outre la réalisation d'une deuxième analyse spectroscopique d'un échantillon de sérum de sang séché pour obtenir la deuxième signature spectroscopique.

9. Procédé selon la revendication 7, dans lequel la deuxième signature spectroscopique est obtenue en séchant digitalement la signature spectroscopique.

10. Procédé selon la revendication 7, dans lequel un traitement ultérieur de la signature spectroscopique est réalisé sur l'échantillon de sérum de sang liquide, de manière à comparer la signature spectroscopique avec des bases de données de spectres obtenus à partir d'échantillons de sérum de sang liquide provenant de sujets avec des états prolifératifs spécifiques.

11. Procédé selon l'une quelconque des revendications précédentes, dans lequel une analyse de la signature comprend la comparaison et/ou la corrélation de la signature spectroscopique de l'échantillon de sérum de sang liquide avec une ou plusieurs signatures spectroscopiques de référence indicatrices d'une ou de plusieurs maladies prolifératives spécifiques.

12. Procédé selon la revendication 11, dans lequel la signature est corrélée avec un diagnostic et/ou un pronostic favorable ou défavorable basé sur un modèle prédictif développé par un « entraînement » (par ex. via des algorithmes de reconnaissance de formes) d'une base de données d'analyses pré-corrélées.

13. Procédé selon les revendications 11 ou 12, dans lequel l'analyse de la signature fournit une indication si oui ou non un sujet est cancéreux ou non cancéreux et dans lequel l'analyse peut fournir un diagnostic d'un cancer du cerveau primitif ou secondaire.
